# EUROPÄISCHE PATENTSCHRIFT

(11) **EP 1 641 450 B1**
(45) Veröffentlichungstag und Bekanntmachung des Hinweises auf die Patenterteilung: **29.10.2014**
(21) Anmeldenummer: 04740534.5
(22) Anmeldetag: 01.07.2004
(51) Int. Cl.: A61K 31/343, A61K 31/352, A61P 15/16, A61P 15/18

(54) **VERWENDUNG VON INHIBITOREN DER PLASMAMEMBRAN-CALCIUM-ATPASE (PMCA) ZUR HEMMUNG DER SPERMIENMOBILITÄT**
USE OF PLASMA MEMBRANE CALCIUM ATPASE (PMCA) INHIBITORS FOR INHIBITING SPERM MOBILITY
UTILISATION D'INHIBITEURS DE MEMBRANE CELLULAIRE-CALCIUM-ATPASE (PMCA) POUR INHIBER LA MOBILITE DU SPERME

(30) Priorität: 03.07.2003 DE 10330213
(43) Veröffentlichungstag der Anmeldung: 05.04.2006
(73) Patentinhaber: Neyses, Ludwig, Manchester M20 5hj (GB)
(72) Erfinder: Neyses, Ludwig, Manchester M20 5hj (GB)
(74) Vertreter: Grund, Martin
(86) Internationale Anmeldenummer: PCT/EP2004/007168
(87) Internationale Veröffentlichungsnummer: WO 2005/002495

(56) Entgegenhaltungen:
- EP-A- 0 552 108
- WO-A-02/05793
- WO-A1-00/40231
- WO-A2-02/05794
- US-A- 4 826 968
- US-A- 5 854 216
- SCHUH KAI ET AL: "Plasma membrane Ca2+ ATPase 4 is required for sperm motility and male fertility" JOURNAL OF BIOLOGICAL CHEMISTRY, Bd. 279, Nr. 27, 2. Juli 2004 (2004-07-02), Seiten 28220-28226, XP008036550 ISSN: 0021-9258
- DATABASE CA [Online] CHEMICAL ABSTRACTS SERVICE, COLUMBUS, OHIO, US; USPENSKAYA, N. V. ET AL: "Intracavitary marker of adult cutworms" XP002394497 gefunden im STN Database accession no. 1975:588826 & MATER. S'EZDA VSES. ENTOMOL. O-VA., 7TH , VOLUME 2, 157. EDITOR(S): KERZHNER, I. M. PUBLISHER: AKAD. NAUK SSSR, ZOOL. INST., LENINGRAD, USSR. CODEN: 31GRAT, 1974,
- SCHUH KAI ET AL: "Plasma membrane Ca2+ ATPase 4 is required for sperm motility and male fertility" JOURNAL OF BIOLOGICAL CHEMISTRY, Bd. 279, Nr. 27, 2. Juli 2004 (2004-07-02), Seiten 28220-28226, XP008036550 ISSN: 0021-9258
- KANWAR U ET AL: "GOSSYPOL INHIBITION OF CA++ UPTAKE AND CA++-ATPASE IN HUMAN EJACULATED SPERMATOZOAL PLASMA MEMBRANE VESICLES" CONTRACEPTION, GERON-X, INC., LOS ALTOS, CA, US, Bd. 39, Nr. 4, 1. April 1989 (1989-04-01), Seiten 431-445, XP000561575 ISSN: 0010-7824
- BREITBART H ET AL: "THE ROLE OF CALCIUM AND CA2+-ATPASE IN MAINTAINING MOTILITY IN RAM SPERMATOZOA" JOURNAL OF BIOLOGICAL CHEMISTRY. (MICROFILMS), AMERICAN SOCIETY OF BIOLOGICAL CHEMISTS, BALTIMORE, MD, US, Bd. 260, Nr. 21, 25. September 1985 (1985-09-25), Seiten 11548-11553, XP001009929
- PATNI ANIL K ET AL: "Role of intracellular calcium in the spermicidal action of 2',4'-dichlorobenzamil, a novel contact spermicide" JOURNAL OF PHARMACY AND PHARMACOLOGY, Bd. 53, Nr. 10, Oktober 2001 (2001-10), Seiten 1387-1392, XP008036557 ISSN: 0022-3573
- SCHUH KAI ET AL: "The sarcolemmal calcium pump PMCA: An effector of platelet aggregation." CIRCULATION, Bd. 106, Nr. 19 Supplement, 5. November 2002 (2002-11-05), Seiten II-79, XP008036566 & ABSTRACTS FROM SCIENTIFIC SESSIONS; CHICAGO, IL, USA; NOVEMBER 17-20, 2002 ISSN: 0009-7322
- YANG Z ET AL: "Na+-Ca2+ exchange activity is localized in the T-tubules of rat ventricular myocytes" CIRCULATION RESEARCH, Bd. 91, Nr. 4, 23. August 2002 (2002-08-23), Seiten 315-322, XP008036552 ISSN: 0009-7330
- BASSANI R A ET AL: "RELAXATION IN FERRET VENTRICULAR MYOCYTES: ROLE OF THE SARCOLEMMAL CA ATPASE", PFLUEGERS ARCHIV: EUROPEAN JOURNAL OF PHYSIOLOGY, SPRINGER VERLAG, BERLIN, DE, vol. 430, no. 4, 1 January 1995 (1995-01-01), pages 573-578, XP008036581, ISSN: 0031-6768, DOI: 10.1007/BF00373894
- QI-XIAN S ET AL: "SPERMINE INHIBITION OF IN VITRO FERTILIZING ABILITY OF HUMAN SPERMATOZOA AND ITS POSSIBLE MODE OF ACTION", SHENGLI XUEBAO - ACTA PHYSIOLOGICA SINICA, ZHONGGUO YIKE DAXUE XUEBAO BIAN-WEI-HUI, BEIJING, CN, vol. 43, no. 5, 1 January 1991 (1991-01-01), page 488, XP008036465, ISSN: 0371-0874
- HOLMES M E ET AL: "MECHANISM OF ACTION OF THE NOVEL PLASMA MEMBRANE CA2+-PUMP INHIBITOR CALOXIN", CELL CALCIUM (EDINBURGH), CHURCHILL LIVINGSTONE MEDICAL JOURNALS, EDINBURGH, GB, vol. 33, no. 4, 1 April 2003 (2003-04-01), pages 241-245, XP008036462, ISSN: 0143-4160, DOI: 10.1016/S0143-4160(02)00207-5
- JONES D M ET AL: "IMMOBILIZATION OF SPERM BY CONDOMS AND THEIR COMPONENTS", CLINICAL REPRODUCTION AND FERTILITY, BLACKWELL SCIENTIFIC PUBLICATIONS, MELBOURNE, AU, vol. 4, no. 6, 1 December 1986 (1986-12-01), pages 367-372, XP008036469, ISSN: 0715-556X
- BURETTE ALAIN ET AL: "Isoform-specific distribution of the plasma membrane Ca2+ ATPase in the rat brain", JOURNAL OF COMPARATIVE NEUROLOGY, WILEY-LISS, NEW YORK, NY, US, vol. 467, no. 4, 22 December 2003 (2003-12-22), pages 464-476, XP008150404, ISSN: 0021-9967
- STRID HILJA ET AL: "ATP-dependent Ca2+ transport is up-regulated during third trimester in human syncytiotrophoblast basal membranes", PEDIATRIC RESEARCH, WILLIAMS AND WILKINS, BALTIMORE, MD, US, vol. 48, no. 1, 1 July 2000 (2000-07-01), pages 58-63, XP008150403, ISSN: 0031-3998

## Beschreibung

Die vorliegende Erfindung bezieht sich auf die Verwendung von Inhibitoren der Plasmamembran-Calcium-ATPase (PMCA) zur Hemmung der Spermienmobilität zur Erzielung einer empfängnisverhütenden Wirkung gemäss der Anspruchen. Weiterhin betrifft die Erfindung Empfängnisverhütungsmittel, die einen oder mehrere PMCA-Inhibitoren enthalten.

### GEBIET DER ERFINDUNG

Je nach sozialen, geographischen und religiösen/ ethischen Gegebenheiten werden zur Zeit folgende verschiedene Verfahren zur Empfängnisverhütung mit unterschiedlicher Zuverlässigkeit angewandt: 1) Kontrazeption ohne Anwendung mechanischer oder chemischer Mittel ("natürliche Verfahren"); 2) Kontrazeption durch Barriereverfahren, dazu zählen die Anwendung von Präservativen, Okklusivpessar und Scheidendiaphragma; 3) Kontrazeption durch lokal wirkende Substanzen, in der Regel Spermizide; 4) Hormonale Kontrazeption bei der Frau durch regelmäßige Einnahme östrogen- und/oder gestagenhaltiger Präparate nach einem bestimmten Schema; 5) Intrauterine Kontrazeption mittels Intrauterinpessar und 6) Operative Sterilisation des Mannes bzw. der Frau.

Die hormonale Kontrazeption stellt hierbei mit großem Abstand das am weitesten verbreitete Verfahren dar. Es ist zuverlässig in der Verhütung von Schwangerschaften und meist reversibel, d.h., nach Absetzen der Präparate aufgrund von Kinderwunsch kommt es in der überwiegenden Zahl der Fälle zur Schwangerschaft. Ungünstigerweise sind jedoch auch zahlreiche absolute und relative Kontraindikationen der hormonalen Kontrazeption bekannt. Zu den absoluten Kontraindikationen der hormonalen Kontrazeption gehören Thrombosen, Embolien, thromboembolische Erkrankungen, akute und progradiente Lebererkrankungen, hormonabhängige maligne Tumoren, Störungen der Gallensekretion, Sichelzellanämie, Diabetes mellitus mit Gefäßschäden, schwer einstellbare Hypertonie und andere Faktoren. Beispiele für relative Kontraindikationen sind Niereninsuffizienz, Herzinsuffizienz, Raynaud-Syndrom, periphere Durchblutungsstörungen, Ödeme, Nikotinkonsum und andere.

Im Vergleich zur hormonalen Kontrazeption sind Verfahren der "natürlichen Kontrazeption" zwar meist nicht kontraindiziert, jedoch sehr unzuverlässig und auch durch eine unakzeptable hohe Versagerquote gekennzeichnet.

Mechanische Verfahren wie Pessare und Sterilisation weisen ebenfalls erhebliche Nachteile aufgrund höherer Komplikationsraten (Pessare) bzw. wegen ihrer weitgehenden Irreversibilität (Sterilisation) auf.

Zusammengefasst lässt sich daher feststellen, dass die herkömmlichen Verfahren der Empfängnisverhütung unter erheblichen Nachteilen leiden. Ein weiterer nachteiliger Faktor besteht darin, dass das sichere Verfahren zur Empfängnisverhütung, die hormonale Kontrazeption, bisher erfolgreich nur von der Frau angewandt werden kann, wobei jedoch die generell verbreitete Auffassung darin besteht, dass die Empfängnisverhütung nicht nur eine zentrale Aufgabe der Frau, sondern beider Partner ist.

Dieser Erfindung liegt daher die Aufgabe zugrunde, neuartige nicht-hormonalen Verhütungsmittel als oral oder parenteral zu applizierende Präparate oder spermizide Substanzen bereitzustellen, die sowohl von der Frau als auch vom Mann angewandt werden können.

Die Erfindung wird durch die folgenden Abbildungen veranschaulicht.

### KURZE BESCHREIBUNG DER ABBILDUNGEN

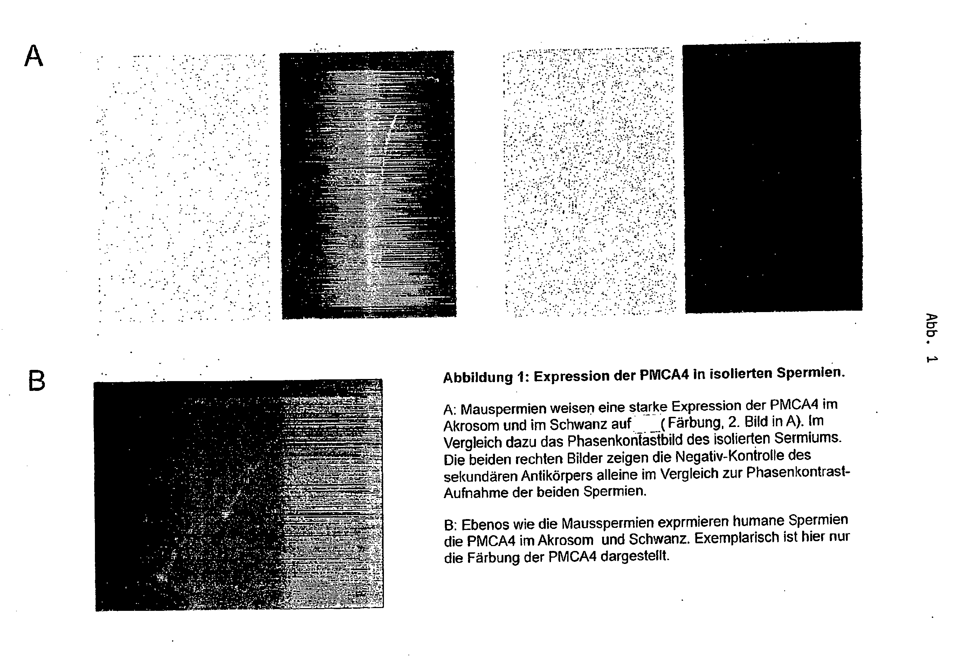

**Abb. 1****:** Expression der PMCA4 im Spermium. ***A*.** Mausspermien weisen eine starke Expression der PCMA4 im Akrosom und im Schwanz auf (rote Färbung, 2. Bild in A). Im Vergleich dazu das Phasenkontrastbild des isolierten Spermiums. Die beiden rechten Bilder zeigen die Negativ-Kontrolle des sekundären Antikörpers alleine im Vergleich zur Phasenkontrast-Aufnahme der beiden Spermien. ***B***. Ebenso wie die Mausspermien exprimieren humane Spermien die PMCA4 im Akrosom und Schwanz. Exemplarisch ist hier nur die Färbung der PCMA4 dargestellt.

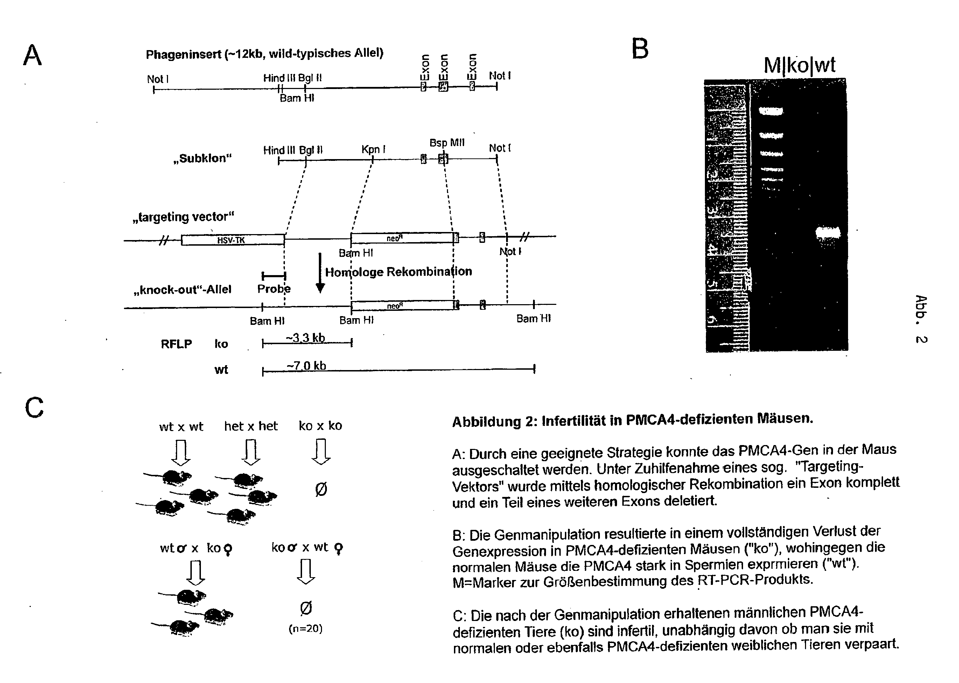

Abb. 2: Fertilität in PMCA4-defizienten Mäusen. A. Durch eine geeignete Strategie konnte das PMCA4-Gen in der Maus ausgeschaltet werden. Unter Zuhilfenahme eines sog. "Targeting-Vektors" wurde mittels homologer Rekombination ein Exon komplett und ein Teil eines weiteren Exons deletiert. ***B**.* Die Genmanipulation resultierte in einem vollständigen Verlust der Genexpression in PMCA4-defizienten Mäusen ("ko"), wohingegen die normalen Mäuse die PCMA4 stark in Spermien exprimieren ("wt"). M = Marker zur Größenbestimmung des RT-PCR-Produkts. ***C***. Die nach der Genmanipulation erhaltenen männlichen PMCA4-defizienten Tiere (ko) sind infertil, unabhängig davon, ob man sie mit normalen oder ebenfalls PMCA4-defizienten weiblichen Tieren verpaart.

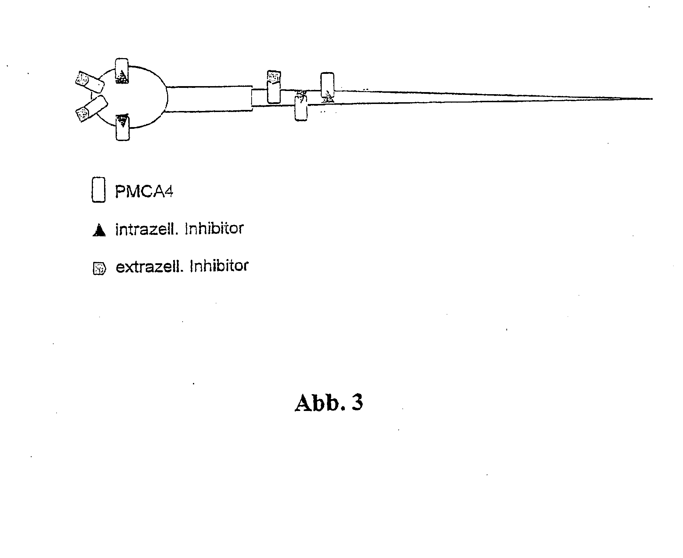

Abb. 3: Schematische Darstellung der Wirkungsweise der PMCA-Inhibitoren. Extrazellulär an die PMCA (weiss) bindende Inhibitoren (grau dargestellt) wirken direkt, während intrazellulär wirkende Substanzen (schwarz) die Zellmembran passieren müssen und dort ihre Wirkung entfalten können.

### AUSFÜHRLICHE BESCHREIBUNG DER ERFINDUNG

Die Erfindung wird durch die beigefügten Ansprüche bestimmt. Die vorliegende Erfindung betrifft die Verwendung von Inhibitoren der Plasmamembran-Calcium-ATPase (PMCA) zur Hemmung der Spermienmobilität, wodurch eine empfängnisverhütende Wirkung erzielt wird.

Offenbart sind weiterhin Empfängnisverhütungsmittel, die einen oder mehrere PMCA-Inhibitoren enthalten. Diese Empfängnisverhütungsmittel können zusätzlich zu den PMCA-Inhibitoren weiterhin konventionelle Empfängnisverhütungsmittel enthalten.

Offenbart ist weiter ein Verfahren zur Empfängnisverhütung, wobei ein Inhibitor der Plasmamembran-Calcium-ATPase an einen Säuger verabreicht wird.

Erfindungsgemäß werden zur Erzielung einer empfängnisverhütenden Wirkung ein oder mehrere Inhibitoren der Plasmamembran-Calcium-ATPase verwendet. Dieses Enzym transportiert Kalzium aus dem Zytosol in das Extrazellulärmilieu und weist Homologie zu einigen anderen ATPasen auf, einschließlich der SERCA ATPase und der Natrium/K+-Pumpe. PMCA wird von vier verschiedenen Genen (PMCA1-4) kodiert und Untersuchungen zeigten die Existenz mehrerer Isoformen der PMCA auf, die durch alternatives Splicing der primären Genprodukte erzeugt werden. Während die Genprodukte 1 und 4 in den meisten Geweben transkribiert werden, werden die Produkte 2 und 3 eher gewebespezifisch exprimiert. Die Transkription der Splice-Varianten ist ebenfalls gewebespezifisch. So exprimieren z.B. die ß-Zellen des Pankreas lediglich die 4b Isoform, wobei die alpha- und gamma-Zellen sowohl die 4a, als auch die 4b Isoformen exprimieren. Es konnte ebenfalls nachgewiesen werden, dass unterschiedliche Splice-Varianten unterschiedliche Affinitäten für Kalzium und Calmodulin haben.

Die vorliegende Erfindung beruht auf der Beobachtung, dass männliche Mäuse, die für die Isoform 4 der murinen PMCA defizient sind (sog. "knock-out" Mäuse) infertil sind. Die Tiere sind ansonsten vital und entwickeln sich normal. Weiterhin kann bei diesen Tieren keine erektile Dysfunktion beobachtet werden. Überraschenderweise konnte jedoch nachgewiesen werden, dass die Mobilität der Spermien der PMCA4-"knockout"-Mäuse soweit eingeschränkt ist, dass keine Befruchtung der Eizelle stattfinden kann und die Tiere somit nicht fähig sind, sich fortzupflanzen. Es handelt sich somit um einen hochspezifischen, auf die Spermien beschränkten Effekt

In menschlichen Spermien wird PMCA4 ebenfalls exprimiert. Die mit den PMCA4-"knockout"-Mäusen erhaltenen Ergebnisse zeigen, dass menschliche Spermien, durch die Verwendung von PMCA-Inihitoren ebenfalls so in ihrer Mobilität eingeschränkt werden können, dass keine Befruchtung der weiblichen Eizelle mehr möglich ist.

Die spezifische physiologische Bedeutung der PMCA für die Spermienfunktion ist bis jetzt unbekannt. Weiterhin gab es bis jetzt keine Hinweise, dass die Verwendung von PMCA-Inhibitoren die Mobilität der Spermien hemmt. Die Verwendung von PMCA-Inhibitoren zur Hemmung der Spermienmobilität zur Erzielung einer empfängnisverhütenden Wirkung wird somit hier zum ersten Mal gezeigt.

Die empfängnisverhütende Wirkung, d.h. die Verhinderung der Verschmelzung eines Spermiums mit einer Eizelle beruht auf der Hemmung der Spermienmobilität durch PMCA-Inhibitoren, die dazu führt, dass die Spermien die Eizelle nicht mehr befruchten können.

Die empfängnisverhütende Wirkung wird erfindungsgemäß durch die Verwendung von PMCA-Inhibitoren erzielt.

Der Inhibitor ist gegen eine der vier Isoformen der Plasmamembran-Calcium-ATPase PMCA gerichtet, nämlich gegen die Isoform PMCA4.

Die aus dem Stand der Technik bekannten Inhibitoren der PMCA lassen sich in intrazellulär und extrazellulär wirkende Inhibitoren einteilen. Erfindungsgemäß wird die Verwendung entweder eines extrazellulären oder eines intrazellulären Inhibitors alleine als auch in Kombination beabsichtigt. Dabei können Kombinationen verwendet werden, die entweder mehrere intrazelluläre bzw. "mehrere extrazelluläre Inhibitoren enthalten als auch Kombinationen, die intrazelluläre als auch extrazelluläre Inhibitoren umfassen.

Bekannte Inhibitoren der PMCA, die zur Verwendung in der vorliegenden Erfindung geeignet sind, sind *5-(und-6)-Carboxyeosindiacetat-succinimidylester* (ein Zellmembran-gängiger Inhibitor der PMCA), und das exträzellulär auf die PMCA wirkende Peptid Caloxin 2A1.

In einer Ausführungsform der Erfindung werden *5-(und-6)-Carboxyeosindiacetat-succinimidylester* (150347-60-7 / 2,6-Pyrrolidinedione, 1-[[[3',6'-bis(acetyloxy)-2',4',5',7'-tetrabromospiro [isobenzofuran-1(3H),9'-[9H]xanthen]-5(or 6)-yl]carbonyl]oxy]) oder Derivate davon als Inhibitor der PMCA in Spermien verwendet.

Verwendete Derivate dieses Inhibitors sind die entsprechenden Eosin- und Fluorescein-Derivate (Gatto, C. & Milanik, M. A. (1993), American Journal of Physiology 264, C1577-1586).

Von dem Zellmembran-gängigen Inhibitor *5-(und-6)-Carboxyeosindiacetat-succinimidylester* existieren schon verschiedenste Eosin- und Fluorescein-, Derivate und deren Salze sowie dem *5-(und-6)-Carboxyeosindiacetat-succinimidylester* entsprechende Esterverbicadungen, wie z.B. 5-(und-6)-carboxyfluorescein-succinimidylester (5(6)-FAM). Die Verwendung dieser Derivate stellt ebenfalls eine bevorzugte Ausführungsform der Erfindung dar.

Eine weitere Ausführungsform der Erfindung bezieht sich auf die Verwendung des PMCA-Inhibitors Caloxin 2A1 (Holmes, M. E. et al., (2003). Cell Calcium 33, 241-245; Chaudary J. et al., (2001), Am J Physiol Cell Physiol 280, C1027-1030).

Caloxin 2A1, ein Polypeptid mit der Aminosäuresequenz VSNSNWPSFPSSGGG, wirkt extrazellulär auf die PMCA. Es konnte *in vitro* gezeigt werden, dass dieses Oligopeptid extrazellulär an die PMCA bindet und die Funktion der PMCA inhibitorisch beeinflusst.

Die Ausgangssubstanzen bieten vielfältige Möglichkeiten zur chemischen Modifikation, wodurch die Bindungsaffinität an die PMCA und somit die inhibitorischen Eigenschaften moduliert und verstärkt werden können.

Alle chemischen Modifikationen der Inhibitoren sind denkbar, solange die hemmende Wirkung der Derivate der Inhibitoren auf die Mobilität der Spermien noch erhalten bleibt. Die Mobilität der Spermien wird wie in Beispiel 3 der vorliegenden Anmeldung beschrieben bestimmt.

Die PMCA-Inhibitoren können auf verschiedene Weise verabreicht werden. Die Inhibitoren werden oral oder parenteral verabreicht.

Die Verabreichungsdauer der PMCA-Inhibitoren kann variabel sein. Eine Ausführungsform der Erfindung ist darauf gerichtet, die Inhibitoren zur einmaligen Kontrazeption zu versenden. Die Inhibitoren können jedoch auch mehrmals oder chronisch verabreicht werden. Die Dosierung der PMCA-Inhibitoren kann vom Fachmann mittels der im Stand der Technik bekannten Verfahren leicht ermittelt werden.

Die Verabreichung erfolgt an einen Säuger, vorzugsweise an einen Menschen. In einer bevorzugten Ausführungsform wird ein PMCA-Inhibitor an einen Mann verabreicht. Die Verabreichung von PMCA-Inhibitoren an den Mann zur Empfängnisverhütung beruht daher auf einem völlig neuen Prinzip. Sie greift nicht in die Entwicklung der Spermien ein und hat deshalb auch nicht die Nebenwirkungen, die von Hormonpräparaten für Männer bekannt sind, wie dauerhafte Unfruchtbarkeit, "Verweiblichung" des Körpers, u.a. unerwünschte Nebenwirkungen.

In einer weiteren bevorzugten Ausführungsform wird ein PMCA-Inhibitor auch an eine Frau verabreicht. Reichem sich die PMCA-Inhibitoren im Mukus (Schleim) des Grebärmuttermundes oder in der Gebärmutter an, so wird beim Geschlechtsverkehr das Sperma, sobald es eintritt, in seiner Mobiltät gehemmt und daher ein empfängnisverhütender Effekt erzielt.

Bevorzugt wird ein PMCA-Inhibitor sowohl an den Mann als auch an die Frau verabreicht, um den größtmöglichsten empfängnisverhütenden Effekt zu erzielen.

Offenbart ist weiterhin auch ein Empfängnisverhütungsmittel, das einen PMCA-Inhibitor in Kombination mit einem pharmazeutisch verträglichen Träger enthält. Bevorzugt enthält das Empfängnisverhütungsmittel nicht nur einen PMCA-Inhibitor, sondern eine Kombination von weinigstens zwei PMCA-Inhibitoren in Kombination mit einem pharmazeutisch verträglichen Träger.

Als pharmazeutisch verträglicher Träger können die üblichen festen oder flüssigen Trägerstoffe oder Verdünnungsmittel und die üblicherweise verwendeten pharmazeutischtechnischen Hilfsstoffe entsprechend der gewünschten Applikationsart mit einer geeigneten Dosierung verwendet werden.

Offenbarte Empfängnisverhütungsmittel können auch in Kombination mit konventionellen Verhütungsmitteln verwendet werden, um Verhütungsmittelzusammensetzungen zu ergeben. Das konventionelle Verhütungsmittel kann dabei ausgewählt werden aus der Gruppe bestehend aus Kondomen, Diaphragmen, Verhütungsschatun etc.

So kann z. B. ein offenbartes Empfängnisverhütungsmittel zur Beschichtung eines Kondoms verwendet werden, um ein verbessertes konventionelles Verhütungsmitteln mit verbesserter Verhütungswirkung zu erhalten. Wird Sperma mit derart beschichteten Verhütungsmitteln in Kontakt gebracht, wird die Spermienmobilität gehemmt und ein empfängnisverhütender Effekt erzielt.

Weiterhin ist die Erfindung auch auf die molekulare Untersuchung der PMCA, insbesondere des PMCA-kodierenden Gens gerichtet. Dies umfasst auch die Charakterisierung der PMCA-Isoformen und Splice-Varianten, als auch den Nachweis von Mutationen und anderen Veränderungen, wie z.B. posttranslationeller Veränderungen zur Diagnostik der Infertilität beim Mann.

Infertilität (d.h. Kinderlosigkeit trotz Kinderwunsch und normalem Geschlechtsverkehr) besteht bei etwa 15% aller Ehepaare. Bei ca. 40% dieser Fälle liegt die Ursache für die Infertilität beim männlichen Partner. Die Diagnostik dieses Leidens ist noch sehr unvollständig und insbesondere sind die genetischen Defekte, die zugrundeliegen können, nur in Ansätzen bekannt.

Da die PMCA-Isoform 4 auch in menschlichen Spermien exprimiert wird, liegt es in Analogie zum Mausmodell nahe, dass auch im menschlichen Organismus ein die PMCA4 betreffender Gendefekt Auswirkungen auf die Fertilität des Mannes hat. Eine Ausführungsform der Erfindung ist daher auf diagnostische Verfahren gerichtet, die den Nachweis umfassen, ob männliche Infertilität auf mangelnder Expression der PMCA mRNA oder einem Gendefekt des PMCA4-Gens beruht.

Das Expressionsniveau, sowie eventuelle Mutationen oder Polymorphismen im PMCA4-Gen und in den Genen, die für die anderen PMCA-Isoformen kodieren, können in Spermien im Ejakulat des Mannes mittels PCR und eventuell sich anschließender Sequenzierung, ELISA, Immunhistologischen Nachweis, Western Blot und PMCA-Aktivitätsbestimmung bestimmt werden. Die Aktivitätsbestimmung der PMCA erfolgt dabei anhand aus dem Stand der Technik bekannten Verfahren. Ergebnisse dieser Untersuchungen können als Ausgangsbasis zur Entwicklung geeigneter Therapien zur Behandlung der männlichen Infertilität entwickelt werden..

Die nachfolgenden Beispiele sind lediglich zur Veranschaulichung der Erfindung bestimmt und beschränken den Schutzumfang der Erfindung nicht. Beispiele, die nicht unter den Schutzumfang der Ansprüche fallen, dienen um der Veranschaulichung.

### BEISPIELE

### Etablierung der PMCA4-defizienten Mauslinie

Aus zwei verschiedenen (heterozygot PMCA4-defizienten) embryonalen Stammzellklonen wurden Mauslinien verstellt, deren PMCA4-Gen durch homologe Rekombination deletiert wurde. Es wurde ein Bereich des Gens deletiert, der das Exon 2 sowie Teile des Exons 3 enthielt (Abb. 2). Durch die Einführung der Neomycinresistenz-Kasette wurden außerdem multiple Stop-Codons in allen drei Leserastern eingeführt, wodurch eine eventuelle Expression eines aberranten Proteins ("Durchleseprodukt" oder *"Splicing"*-Varianten) ausgeschlossen wurde. Insgesamt wurden 14 männliche und 6 weibliche Chimären geboren. Die männlichen Tiere wurden mit C57B1/6-Tieren verpaart. Bei mehreren chimären Mäusen konnte die Integration des zerstörten Allels in die Keimbahn nachgewiesen werden. Die männlichen PMCA-defizienten Tiere waren infertil, während weiblichen Mäuse eine normale Fertilität aufwiesen.

### Expressionanalyse der PMCA4 in murinen und menschlichen Spermien

Die Expression der PMCA4 in murinen und menschlichen Spermien wurde immunhistochemisch nachgewiesen. Die Ergebnisse sind in Abb.1 dargestellt. Teil A von Abb. 1 zeigt, dass Mausspermien eine starke Expression der PCMA4 im Akrosom und im Schwanz aufweisen (rote Färbung, 2. Bild in A). Im Vergleich dazu das Phasenkontrastbild des isolierten Spermiums. Die beiden rechten Bilder zeigen die Negativ-Kontrolle des sekundären Antikörpers alleine im Vergleich zur Phasenkontrast-Aufnahme der beiden Spermien. Teil B von Abb. 1 zeigt, dass humane Spermien ebenso wie Mausspermien die PMCA4 im Akrosom und Schwanz exprimieren. Exemplarisch ist hier nur die Färbung der PCMA4 dargestellt.

### Hemmung der Spermienmobilität durch PMCA-Inbibitoren

Die Hemmung der Funktionalität der Spermien, d.h. die Hemmung der Mobilität wurde wie folgt bestimmt:
PMCA4-defiziente männliche Mäuse wurden getötet und die Nebenhoden präpariert. Diese wurden in 2 ml Spermien-Präparationsmedium ("Sperm Preparation Medium" der Firma MediCult a/s, Möllehaven 12, 4040 Jyllinge, Denmark, Komponenten: Earle's balancierte Salzlösung, Natriumpyruvat, synthetischer Serumersatz, HEPES, Natriumbicarbonat, menschliches Serumalbumin; Penicillin 50,000 IU/Liter und Streptomycin 50 mg/Liter; Kahn, J.A., Human Reproduction 8/9, Seiten 1414-1419, 1993; Lähteenmäki, A., Human Reproduction 10/1, Seiten 142 147, 1995) gebracht und darin mit einem sterilen Skalpell angeritzt. Den funktionierenden Spermien wurde 15 min Zeit gegeben aus dem Nebenhoden heraus zu schwimmen, der Spermien enthaltende Überstand wurde abgenommen und mit jeweils 500 µl steigenden Konzentrationen 5- und 6-Carboxyeosindiacetat-succinimidylester (CE) versetzt. Danach wurden die Sperznien 1 h bei 37°C inkubiert. Während dieser Zeit diffundiert CE in die Spermien und der Ester wird intrazellulär hydrolysiert. Anschließend wurden die sich bewegenden Spermien in Relation zu den unbeweglichen ausgezählt und folgende Ergebnisse erzielt:

**Tabelle I**

| **Hemmung der Spermienmobilität durch 5- und 6-CE-succinimidylester (CE)** | | | | |
|---|---|---|---|---|
| **Konzentration CE (µm)** | - | 5 µM | 20µM | 100µM |
| **Unbewegliche Spermien** | 30%^{a} | 90% | 95% | 97% |

| | | | | |
|---|---|---|---|---|
| ^{a} Die Rate von etwa 30% unbeweglichen Spermien in Abwesenheit von Inhibitor ist bei Präparationen aus dem Nebenhoden normal | | | | |

Die Hemmung der Spermienmobilität wurde weiterhin mit dem CASA System 4.2 der Firma medeaLAB, Erlangen, Deutschland, automatisiert ausgewertet. Die Präparation der Spermien und die Zugabe des Inhibitors erfolgte dabei wie vorstehend beschrieben. Mit dem CASA-System wurden die folgenden drei Gruppen untersucht:
Gruppe A (unbehandelte, normale Spermien, 45 min nach Präparation, 37°C); Gruppe B (PMCA-defiziente Spermien, 45 min nach Präparation, 37°C); Gruppe C (normale Spermien, 45 min nach Präparation, 37°C, davon 30 min mit 20 µM des intrazellulären Inhibitors 5-und 6-CE).

Bei dieser Messung wurden die folgenden Ergebnisse erzielt:
Gruppe A: Progressive Mobilität 73%, Ortsbeweglich bzw. immotil 27 %, n= 254 Spermien;
Gruppe B: Progressive Motilität 14 %, Ortsbeweglich bzw. immotil 86 %, n= 198 und Gruppe C: Progressive Mobilität 22 %, Ortsbeweglich bzw. immotil 78 %, n=217.

Dabei ist zu beachten, dass die Inkubationszeit (gegenüber der manuellen Auszählung) verkürzt war (nur 30 min statt 1 h) und das die Software auch irrelevante durchschwimmende Objekte erfasst, die bei manueller Auszählung ausgeschlossen werden können.

## Patentansprüche

1. Orale oder parenterale Zusammensetzung, welche ein Inhibitor der Plasmamembran-Calcium-ATPase (PMCA) enthält zur Hemmung der Spermienmobilität zur Bewirkung der Empfängnisverhütung,
wobei der Inhibitor gegen die Isoform PMCA4 gerichtet ist,
wobei der Inhibitor 5- oder 6-Carboxyeosindiacetat-succinimidylester oder ein Eosin- oder Fluoresceinderivat davon ist, oder
wobei der Inhibitor Caloxin 2A1 ist.

2. Zusammensetzung gemäß den Ansprüch 1, wobei die Inhibitoren einmaligen oder chronisch verabreicht werden.

3. Zusammensetzung gemäß den Ansprüchen 1 or 2, wobei die Inhibitoren an einen Säuger, vorzugsweise an einen Menschen verabreicht werden.

4. Zusammensetzung gemäß den Ansprüchen 1-3, wobei der PMCA4-Inhibitor in Kombination mit einem pharmazeutisch verträglichen Träger verabreicht wird.

5. Ein in vitro Verfahren zur Infertilitätsdiagnostik beim Mann, umfassend:
Bestimmen mangelnder Expression von PMCA4 mRNA oder eines Gendefekts des PMCA4-Gens in Exon 2 oder Exon 3 in Spermien im Ejakulat eines Mannes mittels PCR und eventuell sich anschließender Sequenzierung, ELISA, immunhistologischem Nachweis, Western Blot und PMCA-Aktivitätsbestimmung.

## Claims

1. Oral or parenteral composition comprising an inhibitor of the Plasma Membrane Calcium ATPase (PMCA) for inhibition of sperm motility for effecting contraception,
wherein the inhibitor is directed against the isoform PMCA4,
wherein the inhibitor is 5- or 6-carboxyeosin diacetate succinimidyl ester or an eosin or fluorescein derivative thereof, or
wherein the inhibitor is Caloxin 2A1.

2. Composition according to claim 1, wherein the inhibitors are administered a single time or chronically.

3. Composition according to claims 1 or 2, wherein the inhibitors are administered to a mammal, preferably to a human.

4. Composition according to claims 1-3, wherein the PMCA-4 inhibitor is administered in combination with a pharmaceutically acceptable carrier.

5. An in vitro method for diagnosis of infertility in men, comprising:
detection of deficient expression of PMCA4 mRNA or of a gene defect of the PMCA4 gene in exon 2 or exon 3 in sperm in the ejaculate of a man via PCR and optional subsequent sequencing, ELISA, immunohistological detection, Western blot and determination of PMCA activity.

## Revendications

1. Composition orale ou parentérale qui contient un inhibiteur de la membrane plasmique calcium-ATPase (PMCA) pour inhiber la mobilité des spermatozoïdes, ayant pour effet la contraception,
dans laquelle l'inhibiteur est dirigé contre l'isoforme PMCA4,
dans laquelle l'inhibiteur est le diacétate de 5- ou 6-carboxyéosine succinimidylester ou un dérivé d'éosine ou de fluorescéine de celui-ci, ou
dans laquelle l'inhibiteur est la caloxine 2A1.

2. Composition selon la revendication 1, dans laquelle les inhibiteurs sont administrés une fois ou sur la durée.

3. Composition selon les revendications 1 ou 2, dans laquelle les inhibiteurs sont administrés à un mammifère, de préférence, à un être humain.

4. Composition selon les revendications 1 à 3, dans lequel l'inhibiteur de la PMCA4 est administré en combinaison avec un véhicule pharmaceutiquement acceptable.

5. Procédé *in vitro* pour le diagnostic d'infertilité chez l'homme, comprenant :
la détermination d'une expression défectueuse de l'ARNm de la PMCA4 ou d'un défaut génique du gène de la PMCA4 dans l'exon 2 ou l'exon 3 dans les spermatozoïdes de l'éjaculat d'un homme au moyen d'une PCR et ensuite, éventuellement, un séquençage, un test ELISA, une détection immuno-histologique, un Western blot et une détermination de l'activité de la PMCA.
